(19) Europäisches Patentamt

European Patent Office

Office européen des brevets

(11) **EP 0 299 490 B2**

(12) **NEUE EUROPÄISCHE PATENTSCHRIFT**

(45) Veröffentlichungstag und Bekanntmachung des Hinweises auf die Entscheidung über den Einspruch:
**13.03.1996 Patentblatt 1996/11**

(51) Int. Cl.$^6$: **A61C 9/00**

(45) Hinweis auf die Patenterteilung:
**06.10.1993 Patentblatt 1993/40**

(21) Anmeldenummer: **88111349.2**

(22) Anmeldetag: **14.07.1988**

(54) **Verfahren zur Herstellung von Zahnersatz**

Method for producing dental prosthesis

Procédé pour la fabrication de dents artificielles

(84) Benannte Vertragsstaaten:
**AT BE CH DE ES FR GB GR IT LI LU NL SE**

(30) Priorität: **16.07.1987 DE 3723555**

(43) Veröffentlichungstag der Anmeldung:
**18.01.1989 Patentblatt 1989/03**

(73) Patentinhaber: **Steinbichler, Hans, Dr.**
**D-83115 Neubeuern (DE)**

(72) Erfinder:
• **Steinbichler, Hans, Dr.**
**D-8201 Neubeuern (DE)**

• **Willer, Jürgen, Dr.**
**D-7410 Reutlingen (DE)**

(74) Vertreter: **Zinnecker, Armin, Dipl.-Ing.**
**Lorenz-Seidler-Gossel,**
**Widenmayerstrasse 23**
**D-80538 München (DE)**

(56) Entgegenhaltungen:
| | |
|---|---|
| EP-A- 0 025 911 | EP-A- 0 040 165 |
| EP-A- 0 054 785 | EP-A- 0 091 876 |
| DE-A- 2 719 696 | US-A- 4 070 683 |
| US-A- 4 575 805 | US-A- 4 611 288 |
| US-A- 4 663 720 | |

EP 0 299 490 B2

## Beschreibung

Die Erfindung betrifft ein Verfahren zur Herstellung von Zahnersatz nach dem Oberbegriff des Anspruchs 1.

Die bisher gebräuchliche Methode, Zahnersatz herzustellen, beruhte darauf, die individuell unterschiedlichen klinischen Mundverhältnisse mit Abdrucken und danach gewonnenen Modellen wiederzugeben. Ein Verfahren zur Herstellung von Zahnersatz muß es ermöglichen, eine hohe Paßgenauigkeit des Zahnersatzes zu erreichen, da diese Paßgenauigkeit Voraussetzung ist für die sichere Verankerung und den dauerhaften Sitz des Zahnersatzes. Alle am Kauvorgang beteiligten Gewebe sind dann in der Lage, die einwirkenden physiologischen Belastungen ohne Schädigung zu kompensieren.

Alle zahnärztlichen und alle sich anschließenden zahntechnischen Arbeitsgänge erfolgen nach seit Jahrzehnten gebräuchlichen Methoden, welche am Beispiel einer Einzelkrone kurz beschrieben werden sollen:

1.) Abdruck des präparierten Zahnstumpfes mit einem gummielastischen oder hydrocolloidalen Abdruckmaterial;
2.) Provisorische Versorgung;
3.) Ausgießen des Abdruckes mit Superhartgips und Herstellen eines Arbeitsmodells sowie eines Gegenbißmodells;
4.) Einbringen der Modelle in einen Artikulator;
5.) Herstellen einer künstlichen Zahnkrone nach Wachsmodellation und Metallguß;
6.) Einpassen des Gußstückes im Mund auf den Zahnstumpf und definitives Eingliedern der künstlichen Krone.

Diese konventionelle Herstellung aus Metall oder anderen Materialien erfordert eine Vielzahl zahnärztlicher und zahntechnischer Abläufe, welche eine große Zahl von Ungenauigkeiten und Fehlermöglichkeiten beinhalten. Paßungenauigkeiten können die Folge sein, die Wiederholungen der Arbeitsabläufe erforderlich machen.

Materialeigenschaften wie Gipsexpansion, Metallschrumpfung und ähnliches sowie unsachgemäße Verarbeitung und schwierige Mundverhältnisse sind dafür verantwortlich zu machen.

Sei einiger Zeit sind Bemühungen im Gange, nach denen unter Verwendung anderer Techniken die konventionelle Abdruckmethode sowie zahlreiche anschließende Arbeitsvorgänge ersetzt werden sollen. Diesen Techniken liegen mechanische oder optische dreidimensionale Meß- und Abtastvorrichtungen zugrunde. Die so gewonnenen Informationen werden gespeichert und an Steuerungsvorrichtungen von bereits in der Werkzeugmaschinenindustrie gebräuchlichen NC-Bearbeitungsmaschinen (numerisch gesteuerten Bearbeitungsmaschinen) weitergegeben.

Die US-PS 4 182 312 zeigt eine mechanische Abtastung dreidimensionaler Oberflächeninformationen von Zähnen und umliegenden Geweben direkt am Patienten. Dabei wird eine Sonde vom Behandler im Munde des Patienten geführt. Eine mechanische Abtastung bringt jedoch gewisse Ungenauigkeiten mit sich.

Die US-PS 3 861 044 beschreibt ein Verfahren, welches die Kavität des Zahnes fotografisch erfaßt. Ein Wachsfüllkörper wird vom Zahnarzt in die gewünschte Endform gebracht.

Auch die im europäischen Patent 0 054 785 beschriebene Methode erfordert konstruktive Korrekturen, bevor die notwendige Paßgenauigkeit erreicht wird. Vorher werden Oberflächen von Körperorganen in ihrer räumlichen und topografischen Gestalt auf optischem Wege berührungsfrei erfaßt.

Das im europäischen Patent EP-0 040 165 aufgezeigte Verfahren versucht, mit Hilfe der holografischen Interferometrie Daten eines präparierten Zahnstumpfes zu erfassen und weiterzuleiten. Mit Laser erzeugtes Licht wird optoelektronisch verarbeitet und einem Rechner zugeführt. Dieses Verfahren wird in der EP-0 040 165 zwar beschrieben; ein derartiges Verfahren konnte jedoch in der Praxis bis heute noch nicht ausgeführt werden.

Aus den Druckschriften US-A-4 663 720 und US-A-4 070 683 sind ein Verfahren zur Herstellung von Zahnersatz bekannt, bei dem Höhenschicht- oder Konturlinien auf dem beschliffenen Zahn und seiner Umgebung erzeugt werden, die Linien mit einer optoelektronischen Einrichtung erfaßt werden, aus den erfaßten Werten die räumliche Struktur des Zahnes und des Zahnersatzes berechnet wird und der Zahnersatz nach an sich bekannten Verfahren gefertigt wird.

Ein Verfahren zur Herstellung von Zahnersatz nach dem Oberbegriff des Anspruchs 1 ist aus der US-A-4 575 805 bekannt. Es werden Konturlinien-Muster erzeugt. Der Abstand der Muster beträgt 1/4 des Konturlinien-Abstandes.

Aufgabe der Erfindung ist es, bei einem Verfahren nach dem Oberbegriff des Anspruchs 1 eine vollautomatische Herstellung des Zahnersatzes zu ermöglichen.

Diese Aufgabe wird durch das in Anspruch 1 definierte Verfahren gelöst.

Wesentlich für die Erfindung ist es, daß Höhenschichtlinien oder Konturlinien auf den Zahnstumpf aufgebracht werden. Die vorliegende Erfindung unterscheidet sich von der in der europäischen Patentschrift EP-0 040 165 beschriebenen Methode grundsätzlich dadurch, daß ihm eine optische Methode zur Erzeugung von Höhenschichtlinien oder Konturlinien zugrundeliegt.

Die Form des beschliffenen Zahnstumpfes, die der Nachbarzähne und der Antagonisten wird durch Konturlinien oder Höhenschichtlinien erfaßt. Die Erzeugung dieser Konturlinien oder Höhenschichtlinien geschieht durch bereits bekannte Verfahren wie beispielsweise Projektionsverfahren oder durch Moire.

Beim Projektionsverfahren werden auf direktem Wege Konturlinien, also Linien, die die äußere Form des Zahnstumpfs beschreiben, aufprojiziert (Bild 1). Die Konturlinien können interferometrisch mit einem Laser oder durch Schattenwurf erzeugt werden. Beim Moire-Verfahren wird der Zahnstumpf durch ein Gitter beleuchtet und durch das gleiche Gitter über eine Fernsehkamera im Abstand von der Lichtquelle beobachtet. Durch Überlagerung des aufprojizierten Schattenmusterns auf dem Zahnstumpf mit dem Gitter entsteht ein Moire, das die Kontur des Zahnstumpfes beschreibt (Bild 2).

Die Konturlinien können mit einer Videokamera erfaßt werden, wobei dies direkt oder über Glasfasern erfolgen kann.

Die Auswertung der Konturlinien erfolgt in einem Bildverarbeitungssystem. Im Gegensatz zu Linienverfolgungs-Programmen wird die Auswertung auf eine Intensitätsmessung zurückgeführt, die über eine Fernsehkamera leicht durchgeführt werden kann. Die Intensität ist im Videosignal für jeden Bildpunkt enthalten.

Die berechnete Kontur läßt sich dann direkt auf eine numerisch gesteuerte Fräsmaschine übertragen, so daß der Zahnersatz paßgenau angefertigt werden kann. Der Zahnersatz kann festsitzend und/- oder herausnehmbar sein.

Das erfindungsgemäße Verfahren bringt den Vorteil mit sich, daß ein Zahnstumpf, die Nachbarzähne und die Antagonisten optisch erfaßt, sofort ausgewertet und die Form des Zahnersatzes berechnet werden kann. Das Verfahren ist tatsächlich ausführbar, einfach und schnell. Die Fertigung des Zahnersatzes erfolgt direkt ohne zeitlichen Zwischenraum und ohne Kontaktaodrücke. Der Zahnersatz kann also während einer Behandlung eingesetzt werden.

Nach der Erfindung werden mindestens drei verschiedene Höhenschicht- oder Konturlinien-Muster erzeugt. Diese Verfahrensweise ermöglicht eine vollautomatische Herstellung des Zahnersatzes.

Der Berechnung der räumlichen Struktur des Zahnes und des Zahnersatzes liegt folgende Formel zugrunde:

$$I = a \times (1 + m \times \cos \Theta) \qquad (1)$$

In dieser Formel bedeuten:
I - Intensität
a - Untergrundhelligkeit
m - Kontrast
$\Theta$ - Winkel

Die Intensität I kann gemessen werden. Sie liegt bei der Aufnahme mit einer Videokamera für jeden Bildpunkt fest. Wenn das Videobild aus 512 x 512 Bildpunkten besteht, kann also diese Intensität für jeden einzelnen der Bildpunkte aus dem Videosignal eindeutig bestimmt bzw. gemessen werden. In der Gleichung verbleiben also drei Unbekannte: die Untergrundhelligkeit a, der Kontrast m und der Winkel $\Theta$. Die gesuchte Größe ist der Winkel $\theta$. Wenn der Winkel $\Theta$ für jeden einzelnen Bildpunkt feststeht, kann daraus auch für jeden einzelnen Bildpunkt die Höhen-Koordinate (z-Koordinate) berechnet werden. Diese Höhen-Koordiante z ist eine Funktion von $\theta$. Mit der Bestimmung der Höhen-Koordinate z für jeden einzelnen Bildpunkt x, y liegt die räumliche Form des Zahnes und des Zahnersatzes eindeutig fest. Ziel ist es also, für jeden einzelnen Punkt x, y die Höhen-Koordinate zu berechnen. Hierzu ist es erforderlich und ausreichend, für jeden einzelnen Bildpunkt x, y den Winkel $\Theta$ zu berechnen. Dies gelingt mit der Gleichung (1) noch nicht, weil diese Gleichung drei Unbekannte beinhaltet. Man benötigt also drei Gleichungen. Um diese drei Gleichungen zu erhalten, verschiebt man zunächst das Höhenschicht- oder Konturlinien-Muster um einen bestimmten Weg, um auf diese Weise ein sich vom ersten Muster unterscheidendes Muster von Höhenschicht- oder Konturlinien zu erhalten. Für dieses zweite Muster gilt dann folgende Gleichung:

$$I(2) = a \times (1 + m \times \cos (\Theta + \Theta(2)) \qquad (2)$$

Der Verschiebewinkel $\Theta(2)$ ist bekannt, da er dem Maß der Linienverschiebung entspricht. Es bleibt also bei den bisherigen drei Unbekannten a, m und $\Theta$. Anschließend wird das Höhenschicht- oder Konturlinien-Muster ein weiteres Mal verschoben. Vorzugsweise betragen die Höhen dieser Verschiebungen ein Viertel bis ein Drittel des Gitterabstandes; es sind aber auch andere (wohldefinierte) Verschiebungen möglich. Die Gleichung für das dritte Muster lautet:

$$I(3) = a \times (1 + m \times \cos (\Theta + \Theta(3)) \qquad (3)$$

Auch der Winkel $\Theta(3)$ ist bekannt, da vorgegeben. Das aus den Gleichungen (1), (2) und (3) entstehende Gleichungssystem ermöglicht jetzt also die Berechung der Unbekannten $\Theta$ und damit die Berechnung der Höhen-Koordinate z. Wenn dieses Verfahren für jeden der Bildpunkte durchgeführt worden ist, ist die gesamte räumliche Struktur berechnet.

Das soeben beschriebene Verfahren der "Phasenverschiebung" bzw. "Linienverschiebung" ermöglicht es, den Zahnersatz vollautomatisch herzustellen. Wenn lediglich ein einziges Höhenschicht-oder Konturlinien-Muster erfaßt und ausgewertet werden würde, müßte zusätzlich noch von Hand eingegeben werden, in welcher Richtung aufsteigende Höhenschichtlinien und in welcher Richtung absteigende Höhenschichtlinien verlaufen. Weiterhin müßte noch eingegeben werden, in welchen Bereichen Schatten oder Fissuren liegen. Nach dem erfindungsgemäßen Verfahren wird mit drei verschiedenen Höhenschicht- oder Konturlinien-Mustern gearbeitet, so daß diese zusätzlichen Informationen nicht mehr eigens eingegeben werden müssen. Nach dem oben beschriebenen Verfahren können dann aus den drei Gleichungen (1), (2) und (3) die z-Koordinaten für jeden Bildpunkt x, y berechnet werden. Eine gesonderte Information über den Anstiegsverlauf der Höhenschichtlinien ist also nicht erforderlich. Die Schatten und die Fissuren können dadurch erkannt werden, daß sie in jedem der drei verschiedenen Musterbilder stets an derselben Stelle liegen.

Die Auswertung der Kontur erfolgt dadurch, daß mindestens drei Bilder in den Rechner eingegeben werden, wobei jeweils das Linienmuster um ein vorherbestimmtes Maß verschoben wird. Aus dem Abstand der Verschiebung und der Intensitätsänderung in jedem Bildpunkt läßt sich die Kontur dann eindeutig berechnen.

Ein Ausführungsbeispiel der Erfindung wird nachfolgend anhand der beigefügten Zeichnungen im einzelnen erläutert. In der Zeichnungen zeigt

Bild 1 die Erzeugung von Konturlinien durch Projektion und

Bild 2 die Erzeugung von Konturlinien durch Moire.

Wie in Bild 1 zu sehen, erzeugt ein Projektor 1 Lichtstrahlen, die auf ein Gitter 2 treffen. Dieses Gitter 2 besitzt lichtundurchlässige Bereiche und waagerechte, lichtdurchlässige Bereiche 3 in einem vorherbestimmten, vorzugsweise gleichen Abstand voneinander. Die Lichtstrahlen verlassen also das Gitter 2 in waagerechten, parallel zueinander verlaufenden Ebenen 4. Sie treffen auf den Zahnstumpf 5 und erzeugen dort Höhenschichtlinien 6. Diese Höhenschichtlinien 6 auf dem Zahnstumpf 5 werden von der Fernsehkamera 7 aufgenommen und an den Monitor 8 sowie an den Computer 9 weitergeleitet. Auf dem Monitor 8 können die Höhenschichtlinien 6 sichtbar gemacht werden, wie mit der Bezugsziffer 10 angedeutet. Der Computer 9 berechnet aus den Intensitätswerten für jeden der Bildpunkte des Monitors die räumliche Struktur des Zahnstumpfs 5. Der Monitor kann beispielsweise aus 512 x 512 Bildpunkten bestehen.

Wenn lediglich ein einziges Bild mit Höhenschichtlinien genommen wird, muß dem Computer noch die Information eingegeben werden, in welcher Richtung die höheren und in welcher Richtung die tieferen Bereiche des Zahnes liegen. Weiterhin muß dem Computer noch die Information eingegeben werden, welche Bereiche Schatten und/oder Fissuren beinhalten.

Um das System vollautomatisch zu machen, ist eine Phasenverschiebung oder Linienverschiebung erforderlich. Zunächst wird ein Bild mit bestimmten Höhenschichtlinien aufgenommen. Dann wird der Projektor 1 senkrecht zur Projektionsachse 11, also senkrecht zu den Ebenen 4, verschoben, und zwar um eine wohldefinierte Höhe, die vorzugsweise etwa ein Viertel bis ein Drittel des Gitterabstandes, also des Abstandes der lichtdurchlässigen Bereiche 3 des Gitters 2 entspricht. Hierdurch wird ein zweites, verschobenes Linienmuster auf dem Zahnstumpf 5 erzeugt und von der Fernsehkamera 7 abgenommen. Dieser Vorgang wird dann ein drittes Mal wiederholt. Auf diese Weise gelangen drei verschiedene Höhenlinienmuster in den Computer 9. Dieser Computer 9 kann daraus die räumliche Zahnstumpfform vollkommen automatisch selbständig berechnen. Die Schatten und/oder Fissuren werden dadurch erkannt, daß sie bei allen drei Aufnahmen an derselben Stelle liegen und sich nicht - wie die Höhenlinien - verschieben. Diese Schatten und/oder Fissuren können also später automatisch bei der Berechnung vom Computer 9 weggelassen werden.

Bild 2 zeigt die Erzeugung von Konturlinien durch Moire. Von der Lichtquelle 21 werden Lichtstrahlen ausgesendet und auf ein Gitter 22 geworfen. Dieses Gitter besteht aus lichtundurchlässigen Bereichen und aus linienförmigen lichtdurchlässigen Bereichen 23. Die linienförmigen lichtdurchlässigen Bereiche sind parallel zueinander angeordnet und verlaufen in einem wohldefinierten, vorzugsweise gleichen Abstand. Die Gitterebene verläuft senkrecht zur Achse 24 des Zahnstumpfs 5. Durch das Gitter werden auf dem Zahnstumpf 5 Konturlinien erzeugt, von denen im Bild 2 lediglich eine einzige Konturlinie 25 gezeigt ist. Die Fernsehkamera 7 befindet sich im Abstand von der Lichtquelle 21. Der Zahnstumpf 5 wird also durch das Gitter 22 beleuchtet und durch das gleiche Gitter 22 über die Fernsehkamera 7 beobachtet. Durch Überlagerung des aufprojizierten Schattenmusters (Konturlinien 25) auf dem Zahnstumpf 5 mit dem Gitter 22 entsteht ein Moire, das die Kontur des Zahnstumpfes 5 beschreibt. Dieses Moire kann auf dem Monitor 8 sichtbar gemacht werden, wie durch die Linien 26 angedeutet. Die Fernsehkamera 7 ist mit dem Monitor 8 und mit dem Computer 9 verbunden.

Die in Bild 2 dargestellte Verfahrensweise unterscheidet sich von derjenigen des Bildes 1 nur dadurch, daß beim Bild 1 Konturlinien durch Projektion erzeugt werden, während beim Bild 2 Konturlinien durch Moire erzeugt werden. Im übrigen gelten also für das Verfahren nach Bild 2 die Ausführungen zum Verfahren nach Bild 1.

**Patentansprüche**

1. Verfahren zur Herstellung von Zahnersatz, bei dem Höhenschicht- oder Konturlinien (6; 25) auf dem beschliffenen Zahn und seiner Umgebung erzeugt werden,
die Linien (6; 25) mit einer optoelektronischen Einrichtung (7) erfaßt werden,
die erfaßten Werte in einen Rechner eingegeben und die räumliche Struktur des Zahnes und des Zahnersatzes berechnet wird, und
anhand der so berechneten Struktur der Zahnersatz nach an sich bekannten Verfahren gefertigt wird,
wobei mindestens drei verschiedene Höhenschicht- oder Konturlinien-Muster erzeugt werden, deren Abstand vorherbestimmt ist und vorzugsweise 1/4 bis 1/3 des Höhenlinien-Abstandes beträgt,
dadurch gekennzeichnet,
daß die räumliche Struktur des Zahnes durch die Formeln

$$I = a \times ( 1 + m \times \cos \Theta ) \qquad (1)$$

$$I(2) = a \times ( 1 + m \times \cos ( \Theta + \Theta(2) ) ) \qquad (2)$$

$$I(3) = a \times ( 1 + m \times \cos ( \Theta + \Theta(3) ) ) \qquad (3)$$

berechnet wird.

**2.** Verfahren nach Anspruch 1, dadurch gekennzeichent, daß die Höhenschicht- oder Konturlinien durch ein Projektionsverfahren (Bild 1) erzeugt werden.

**3.** Verfahren nach Anspruch 1, dadurch gekennzeichnet, daß die Höhenschicht- oder Konturlinien durch ein Moire-Verfahren erzeugt werden (Bild 2).

**4.** Verfahren nach Anspruch 1, dadurch gekennzeichnet, daß die Höhenschicht- oder Konturlinien durch Schattenwurf erzeugt werden.

**5.** Verfahren nach Anspruch 1, dadurch gekennzeichnet, daß die Höhenschicht- oder Konturlinien interferometrisch durch Laser erzeugt werden.

**6.** Verfahren nach einem der vorhergehenden Ansprüche, dadurch gekennzeichnet, daß die Linien (6; 25) mit einer Videokamera (7) erfaßt werden.

**Claims**

**1.** Method for producing a dental prosthesis, in which contour lines (6; 25) are produced on the ground tooth and its surroundings, the lines (6; 25) are detected by an optoelectronic device (7), the detected values are fed into a computer and the spatial structure of the tooth and of the tooth prosthesis is calculated, and from the thus calculated structure the tooth prosthesis is produced using methods known per se, at least three different contour line patterns being produced whose spacing is predetermined and preferably amounts to 1/4 to 1/3 of the contour line spacing; characterized in that the spatial structure of the tooth is calculated by means of the formulae

$$l = a \times ( 1 + m \times \cos \theta ) \qquad (1)$$

$$l\,(2) = a \times ( 1 + m \times \cos (\theta + \theta(2) ) \qquad (2)$$

$$l\,(3) = a \times ( 1 + m \times \cos (\theta + \theta(3) ). \qquad (3)$$

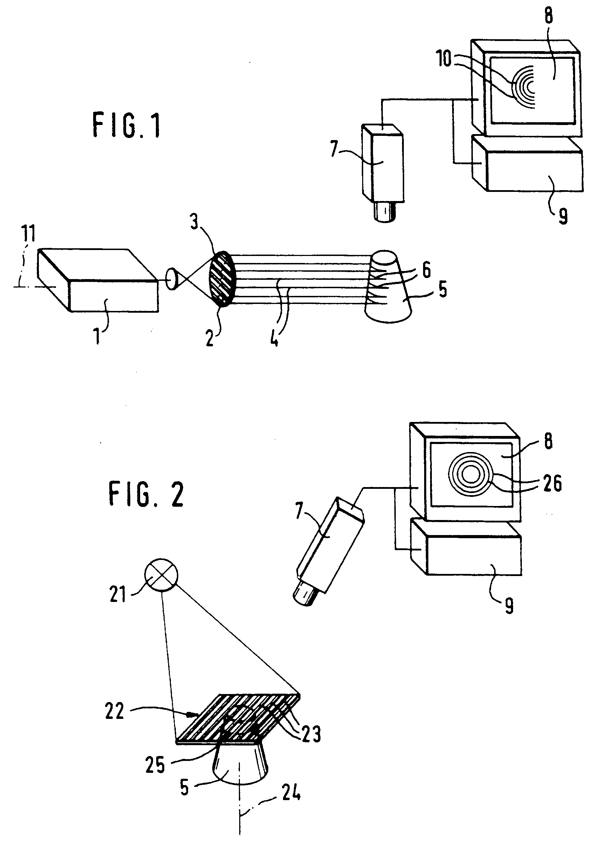

**2.** Method according to Claim 1, characterized in that the contour lines are produced by a projection method (Figure 1).

**3.** Method according to Claim 1, characterized in that the contour lines are produced by a moiré method (Figure 2).

**4.** Method according to Claim 1, characterized in that the contour lines are produced by shadow casting.

**5.** Method according to Claim 1, characterized in that the contour lines are produced interferometrically by laser.

**6.** Method according to one of the preceding claims, characterized in that the lines (6; 25) are detected by a video camera (7).

**Revendications**

**1.** Procédé pour la fabrication de dents artificielles, dans lequel on produit des courbes de niveau ou lignes de contour (6; 25) sur la dent meulée et son environnement, on saisit les lignes (6; 25) avec un dispositif optoélectronique (7), on introduit les valeurs saisies dans un calculateur et on calcule la structure spatiale de la dent et de la dent artificielle et on fabrique à l'aide de la structure ainsi calculée la dent artificielle selon des procédés connus en soi, où au moins trois modèles différents de courbes de niveau ou lignes de contour sont produits dont l'écart est prédéfini et constitue de préférence 1/4 jusqu'à 1/3 de l'écart des courbes de niveau, caractérisé en ce que la structure spatiale de la dent est calculée par les formules

$$l = a \times (1 + m \times \cos \theta) \qquad (1)$$

$$l\,(2) = a \times (1 + m \times \cos (\theta + \theta(2)) \qquad (2)$$

$$l\,(3) = a \times (1 + m \times \cos (\theta + \theta(3)). \qquad (3)$$

**2.** Procédé selon la revendication 1, caractérisé en ce que les courbes de niveau ou lignes de contour sont produites par un procédé de projection (figure 1).

**3.** Procédé selon la revendication 1, caractérisé en ce que les courbes de niveau ou lignes de contour sont produites par un procédé de moirage (figure 2).

**4.** Procédé selon la revendication 1, caractérisé en ce que les courbes de niveau ou lignes de contour sont produites par une projection d'ombre.

**5.** Procédé selon la revendication 1, caractérisé en ce que les courbes de niveau ou lignes de contour sont produites, interférométriquement, par laser.

**6.** Procédé selon l'une des revendications précédentes, caractérisé en ce que les lignes (6;25) sont saisies avec une caméra vidéo (7).

EP 0 299 490 B2

FIG. 1

FIG. 2

6